# EUROPÄISCHE PATENTSCHRIFT

(11) **EP 3 755 289 B1**
(45) Veröffentlichungstag und Bekanntmachung des Hinweises auf die Patenterteilung: **14.05.2025**
(21) Anmeldenummer: 19709380.0
(22) Anmeldetag: 20.02.2019
(51) Int. Cl.: A61F 11/00, A61F 13/40, A61F 13/38

(54) **VORRICHTUNG UND VERFAHREN ZUM REINIGEN DES ÄUSSEREN GEHÖRGANGS**
DEVICE AND METHOD FOR CLEANING THE EXTERNAL AUDITORY CANAL
DISPOSITIF ET PROCÉDÉ POUR NETTOYER UN CANAL AUDITIF EXTERNE

(30) Priorität: 21.02.2018 CH 2102018
(43) Veröffentlichungstag der Anmeldung: 30.12.2020
(73) Patentinhaber: Petignat, Guy, 8703 Erlenbach (CH)
(72) Erfinder: LINDER, Thomas, 6045 Meggen (CH); HENSELER, Mathias, 6048 Horw (CH); MEYER, Andreas, 8125 Zollikerberg (CH); PETIGNAT, Guy, 8703 Erlenbach (CH)
(74) Vertreter: Gachnang, Hans Rudolf
(86) Internationale Anmeldenummer: PCT/EP2019/054167
(87) Internationale Veröffentlichungsnummer: WO 2019/162303

(56) Entgegenhaltungen:
- WO-A1-2015/083161
- CN-U- 204 352 011
- CN-U- 204 468 421
- CN-U- 204 683 905
- CN-U- 206 761 827
- US-A1- 2010 312 198
- US-A1- 2016 287 445

## Beschreibung

Die vorliegende Erfindung betrifft eine Vorrichtung sowie ein Verfahren zum Reinigen des äusseren Gehörgangs, insbesondere zum Entfernen von Cerumen aus dem äusseren Gehörgang.

Zur Reinigung des äusseren Gehörgangs (Meatus acusticus externus) ist die Verwendung sogenannter Wattestäbchen weit verbreitet. Diese bestehen in Regel aus einem Holz-, Papier- oder Kunststoffstäbchen, das wenigstens einen Ends einen Wattebausch aus Cellulose oder Kunststofffasern aufweist. Die Benutzung von Wattestäbchen kann jedoch zu einem ungewollten Vorschieben des Cerumens in Richtung Trommelfell und bei häufiger Anwendung sogar zu dessen Verdichtung bzw. Verfestigung führen. Derart verfestigtes Cerumen ist mitunter nur noch vom Facharzt zu entfernen und bewirkt nicht selten eine signifikante Beeinträchtigung des Hörvermögens.

Aus dem Stand der Technik sind verschiedenartige Vorrichtungen zur Ohrenreinigung bekannt, die diese Problematik verhindern sollen. So beschreibt die Druckschrift US 2010/0312198 A1 eine Reinigungsvorrichtung mit einem radial expandierbaren Reinigungsmittel, das an einem proximalen Endabschnitt eines Instrumentationsstabs angeordnet ist. Das Reinigungsmittel kann beispielsweise ein elastisch verformbares Mittel aufweisen, das sich in einem radial komprimierten Zustand, etwa durch Aufnahme in eine hülsenartige Einführeinrichtung, in den Gehörgang einführen lässt. Durch anschliessendes Freisetzen aus der Einführeinrichtung geht das Reinigungsmittel unter Einwirkung elastischer Rückstellkräfte in einen radial expandierten Zustand über, in welchem es sich bis zur Innenwand des Gehörgangs erstrecken kann. Hierdurch wird beim nachfolgenden Zurückziehen des Reinigungsmittels etwaig in distaler Richtung vorhandenes Cerumen aus dem Gehörgang herausgefördert. Alternativ kann das Reinigungsmittel ein durch Flüssigkeitsaufnahme radial expandierfähiges Material umfassen. Allerdings haben sich derartige Vorrichtungen in der Praxis häufig als schwierig handhabbar erwiesen. Auch sind sie eher aufwendig und teuer in der Herstellung. Zudem werden proximal sehr weit innen gelegene Bereich des äusseren Gehörgangs nur unzureichend gereinigt. In diesem Zusammenhang sei ferner auf das Dokument US2016/0287445 A1 verwiesen.

Aufgabe der vorliegenden Erfindung ist es daher, eine kostengünstige und leicht handhabbare Vorrichtung zur Reinigung des äusseren Gehörgangs anzugeben, die ein Hineinschieben und Verdichten von Cerumen im Gehörgang effektiv verhindert und auch proximal sehr weit innen gelegene Bereiche des äusseren Gehörgangs ausreichend reinigt.

Diese Aufgabe wird durch eine Reinigungsvorrichtung gemäss dem unabhängigen Anspruch gelöst. Vorteilhafte Ausgestaltungen der Erfindung sind Gegenstand der abhängigen Ansprüche.

Gemäss der Erfindung umfasst die Vorrichtung zum Reinigen des äusseren Gehörgangs eine Führungshülse mit einem proximalen Auslassabschnitt zum partiellen Einführen in den äusseren Gehörgang. Die Vorrichtung umfasst ferner einen axial durch die Führungshülse hindurchführbaren Instrumentationsstab. Der Instrumentationsstab weist an einem proximalen Endbereich eine Reinigungseinrichtung mit wenigstens einem umfangsseitig verlaufenden, in radialer Richtung elastisch expandierenden Reinigungsmittel auf. Die Führungshülse und das Reinigungsmittel sind derart ausgebildet, dass ein Innenquerschnitt, insbesondere ein kleinster Innenquerschnitt des proximalen Auslassabschnitts der Führungshülse kleiner ist als ein Aussenquerschnitt, insbesondere ein kleinster Aussenquerschnitt des Reinigungsmittels in einem radial expandierten Zustand. Hierdurch kann das Reinigungsmittel durch Aufnahme bzw. Anordnung des proximalen Endbereichs des Instrumentationsstabs im proximalen Auslassabschnitt der Führungshülse in einen radial komprimierten Zustand überführt werden. In diesem Zustand weist das Reinigungsmittel einen Aussenquerschnitt auf, der kleiner ist als der Innenquerschnitt des Gehörgangs. Letzterer liegt bei erwachsenen Personen typischerweise im Bereich von etwa 4-9 mm, durchschnittlich bei etwa 6-7 mm. Demgemäss weist der Aussenquerschnitt des Reinigungsmittels im radial komprimierten Zustand und daher der Innenquerschnitt, insbesondere der kleinste Innenquerschnitt des proximalen Auslassabschnitts vorzugsweise einen Durchmesser von etwa 2-4 mm auf. Der radial komprimierte Zustand dient folglich in vorteilhafter Weise dazu, das Reinigungsmittel zusammen mit der Führungshülse zumindest partiell in den äussern Gehörgang einzuführen, ohne dass dabei Cerumen durch das komprimierte Reinigungsmittel in proximaler Richtung vorgeschoben oder gar verdichtet wird.

In erfindungsgemässer Weise wurde erkannt, dass die häufig nur unzureichende Reinigung weit innen gelegener Bereich des äusseren Gehörgangs bei vielen aus dem Stand der Technik bekannten Lösungen dadurch bedingt ist, dass diese Reinigungsmittel innerhalb des Gehörgangs beim Überführen aus dem komprimierten in den expandierten Zustand nicht ausschliesslich in radialer Richtung expandieren, sondern gleichzeitig auch eine distal gerichtete Bewegung erfahren. Insbesondere klappen bei einigen bekannten Lösungen die Reinigungsmittel beim Übergang in den expandierten Zustand - etwa beim Zurückziehen einer sie umgebenden Führungshülse - von der Mittenachse des Gehörgangs her in distaler Richtung, d.h. in Richtung Ohrausgang auf. Dabei gelangen die Reinigungsmittel aufgrund der Aufklappbewegung erst in einer im Vergleich zu ursprünglichen Einführtiefe weiter zurückgezogenen Positionen an die Innenwandung des Gehörgangs und entfalten somit ihre reinigende Wirkung nicht bei der ursprünglichen Einführtiefe.

Demgegenüber zeichnet sich die vorliegende Erfindung dadurch aus, dass das wenigstens eine Reinigungsmittel der Reinigungseinrichtung innerhalb des proximalen Auslassabschnitts in einem radial komprimierten Zustand anordenbar ist, in welchem das Reinigungsmittel in distaler Richtung zur Längsachse des Instrumentationsstabs hin umgebogen ist. Hierdurch wird in vorteilhafter Weise bewirkt, dass sich das Reinigungsmittel beim Austritt aus dem proximalen Auslassabschnitt in den radial expandierten Zustand in proximaler Richtung, d.h. zum Ohrinneren hin bzw. in Richtung Trommelfell, aufrichtet. Der Austritt des Reinigungsmittels aus dem proximalen Auslassabschnitt kann bevorzugt durch Vorschieben des Instrumentationsstabs in proximaler Richtung durch die Führungshülse über den proximalen Auslassabschnitt hinaus in den äusseren Gehörgang bewirkt werden. Dabei richtet sich das in distaler Richtung zur Längsachse des Instrumentationsstabs hin umgebogene Reinigungsmittel erst nach vollständigem Austritt aus dem Auslassabschnitt in den expandieren Zustand auf. In vorteilhafter Weise kann hierdurch eine deutlicher grössere Einführtiefe des Reinigungsmittels in proximaler Richtung erzielt werden, ohne dass dabei Cerumen durch das noch komprimierte Reinigungsmittel in proximaler Richtung vorgeschoben oder verdichtet wird. Letzteres wird insbesondere dadurch vermieden, dass das Reinigungsmittel erst bei Erreichen der maximal gewünschten Eindringtiefe des Instrumentationsstabs in den Gehörgang in den radial expandierten Zustand und damit gegebenenfalls in Kontakt mit der Innenwandung des Gehörgangs gelangt. Hierdurch vereinfacht sich die Handhabung und Sicherheit der Reinigungsvorrichtung deutlich.

Definitionsgemäss beziehen sich vorliegend die Begriffe "proximal", "distal" und "radial" auf die Anatomie des äusseren Gehörgangs. Demnach bezeichnet "proximal" eine Richtung zum Ohrinneren bzw. zum Trommelfell hin und "distal" auf eine zum Ohrausgang gewandte Richtung. Mit "radial" wird auf eine quer, insbesondere senkrecht zur Mittenachse des äusseren Gehörgangs verlaufende Richtung Bezug genommen. Entsprechend beziehen sich der proximale Endabschnitt des Instrumentationsstabs und der proximale Austrittsabschnitt der Führungshülse auf jeweils solche Abschnitte, die bei Verwendung der erfindungsgemässen Vorrichtung, insbesondere beim Einführen in den äusseren Gehörgang, dem Ohrinnern bzw. dem Trommelfell zugewandt sind.

Nach einer vorteilhaften Ausgestaltung der Erfindung weist die Reinigungseinrichtung eine Bürste auf. Bürstenartige Reinigungseinrichtungen bzw. -mittel eignen sich in besonderer Weise für die Ohrenreinigung, nicht zuletzt wegen der Flexibilität der Borsten und des damit einhergehenden geringen Verletzungsrisikos. Zudem lassen sich bürstenartige Reinigungseinrichtungen bzw. -mittel besonders einfach durch Umbiegen der Borsten in einen radial komprimierten Zustand überführen. Alternativ können anstelle von Borsten auch flexible Scheiben oder Kreissegmente, beispielsweise aus Kunststoff eingesetzt werden.

Das wenigstens eine Reinigungsmittel der Reinigungseinrichtung kann nach einer besonders vorteilhaften Ausführungsform wenigstens einen umlaufenden Bürstenkranz aus mehreren umfangsseitig angeordneten Borsten aufweisen. Bevorzugt erstrecken sich die Borsten im radial frei expandierten Zustand vom Instrumentationsstab ausgehend quer, insbesondere senkrecht zur Längsachse des Instrumentationsstabs nach aussen. Besonders bevorzugt sind die Borsten eines Bürstenkranzes in einer Axialebene bezüglich der Längsachse des Instrumentationsstabs angeordnet. Insbesondere können die Borsten eines Bürstenkranzes auf einen kreisförmigen Querschnitt angeordnet sein bzw. eine kreisförmige Bürstenscheibe bilden. Bevorzugt weist der Instrumentationsstab zwischen 4 und 10 Bürstenkränze bzw. Bürstenscheiben auf. In einem Bürstenkranz können wenigstens drei, insbesondere wenigstens vier, bevorzugt wenigstens fünf, besonders bevorzugt wenigstens sechs Borsten umfangsseitig um den Instrumentationsstab angeordnet sein. Beispielsweise kann ein Bürstenkranz fünf oder sechs Borsten aufweisen. Die Borsten sind beispielswiese Kunststoffborsten, etwa Borsten aus wenigstens einem Polymer, Polyurethan oder Polypropylen oder Gummi. Der Durchmesser bzw. die Dicke der kann zwischen 0.2 bis 0.6 mm, vorzugsweise 0.35 bis 0.5 mm, betragen. Der Durchmesser der Bürste kann zwischen 8 - 12 mm liegen, vorzugsweise 10 mm. Die Länge der Borsten beträgt bevorzugt 4 - 5 mm. Zudem können die Borsten an den radialen Enden mit Watte beflockt sein. Mehre Borsten können zu einem Borstenbüschel zusammengefasst sein. Mehrere solcher Borstenbüschel können umfangsseitig am proximalen Endabschnitt des Instrumentationsstabs unter Bildung wenigstens eines Bürstenkranzes angeordnet sein.

Die Borsten, flexiblen Scheiben oder Kreissegmente können gleichzeitig mit dem Instrumentationsstab gespritzt werden.

Nach einer weiteren vorteilhaften Ausgestaltung der Erfindung weist der Instrumentationsstab eine Reinigungseinrichtung auf, die mehrere Reinigungsmittel, insbesondere mehrere Bürstenkränze umfasst. Die mehreren Reinigungsmittel, insbesondere mehreren Bürstenkränze sind bevorzugt axial verteilt entlang des proximalen Endabschnitts angeordnet. Hierdurch wird die reinigende Wirkung der Vorrichtung in vorteilhafter Weise erhöht.

Des Weiteren kann der Aussenquerschnitt einer aus mehreren Reinigungsmitteln bestehenden Reinigungseinrichtung - etwa eine Bürste, insbesondere eine Bürste, die mehrere axial voneinander beanstandete Bürstenkränze aufweist - in distaler Richtung abnehmen, vorzugsweise konisch oder stufenartig. So kann eine solche Reinigungseinrichtung eine in distaler Richtung abnehmende, konische oder gestufte Form aufweisen. Dementsprechend können bei einer solchen Ausgestaltung die radialen Abmessungen der mehreren Reinigungsmittel, insbesondere die Durchmesser der Bürstenkränze, mitunter die Längen der Borsten der jeweiligen Bürstenkränze, entlang des proximalen Endabschnitts in distaler Richtung abnehmen. Hierdurch wird erreicht, dass sich sämtliche Reinigungsmittel in den expandierten Zustand erst aufrichten, wenn das entlang des proximalen Endabschnitts proximal zuvorderst angeordnete Reinigungsmittel vollständig aus dem proximalen Austrittsabschnitt ausgetreten ist. In vorteilhafter Weise wird hierdurch die maximale Eindringtiefe des proximalen Endabschnitts bzw. der gesamten Reinigungseinrichtung in den Gehörgang erhöht.

Ferner können die Borsten bei einer Reinigungseinrichtung, die mehrere axial voneinander beanstandete Bürstenkränze aufweist, so angeordnet sein, dass die Borsten benachbarter Bürstenkränze umfangsseitig versetzt zueinander angeordnet sind. Hierdurch wird ebenfalls die Reinigungswirkung erhöht.

Zudem kann es vorgesehen sein, dass die Borsten entlang des proximalen Endabschnitts derart angeordnet sind, dass im komprimierten Zustand mehrere Borsten, vorzugsweise zwei bis drei, übereinander zu liegen kommen. Hierdurch kann in vorteilhafter Weise eine besonders hohe Komprimierung erzielt werden, wodurch die sich Gefahr eines unerwünschten Vorschiebens oder Verdichtens von Cerumen minimiert.

Gemäss einer weiteren vorteilhaften Ausgestaltung der Erfindung kann die Führungshülse, insbesondere der Auslassabschnitt in proximaler Richtung eine hyperbolische, parabolische oder konische bzw. eine hyperbolisch, parabolisch oder konisch ausgebildete Verjüngung des Aussenquerschnitts aufweisen. Beispielsweise kann der proximale Auslassabschnitt einen Aussenkonus aufweisen. Durch die hyperbolische, parabolische oder konische Geometrie der Verjüngung wird erreicht, dass die Eindringtiefe des Auslassabschnitts je nach Weite des Gehörgangs variiert, insbesondere automatisch begrenzt ist und damit das Verletzungsrisiko reduziert wird. Bei schmalen Gehörgängen kann die Führungshülse, insbesondere der Auslassabschnitt weniger tief eindringen als bei weiten Gehörgängen. Zudem wird durch den Aussenkonus ein Anschlag für die Führungshülse beim Ansetzen bzw. partiellen Einführen in den Gehörgang bewirkt, was die Handhabbarkeit der Gesamtvorrichtung erleichtert. Insbesondere kann die Vorrichtung hierdurch einhändig vorgenommen werden.

Zusätzlich zum proximalen Auslassabschnitt kann die Führungshülse einen distalen, vorzugsweise zylinderförmigen Depotabschnitt zur Aufnahme des wenigstens einen Reinigungsmittels im radial expandierten Zustand aufweisen. Dies hat den Vorteil, dass das wenigstens eine Reinigungsmittel zwischen Produktion und Verwendung, etwa während Lagerung und Vertrieb, im radial expandierten, d.h. im entspannten Zustand aufbewahrt wird, wodurch eine Ermüdung der elastischen Materialeigenschaften vermieden werden.

Gleichzeitig wird das Reinigungsmittel von dem umhüllenden Depotabschnitt der Einführhülse vor schädigenden Ausseneinflüssen, etwa Verschmutzungen, geschützt. Der Depotabschnitt der Einführhülse ist dabei so ausgebildet, dass ein kleinster Innenquerschnitt des distalen Depotabschnitts grösser ist als der grösste Aussenquerschnitt des proximalen Endbereichs im radial expandierten Zustand des wenigstens einen Reinigungsmittels.

Gemäss einer weiteren Ausgestaltung der Erfindung kann die Führungshülse, insbesondere der Auslassabschnitt eine Verjüngung des Innendquerschnitts in proximaler Richtung aufweisen, beispielsweise eine hyperbolisch, parabolisch oder konisch ausgebildete Verjüngung des Innendquerschnitts. Insbesondere kann der Auslassabschnitt als Trichter bzw. trichterförmig oder düsenförmig ausgebildet sein. Hierdurch kann in besonders einfacher Weise eine Anordnung des wenigstens einen Reinigungsmittel innerhalb des proximalen Auslassabschnitts im radial komprimierten Zustand erreicht und sichergestellt werden, dass das Reinigungsmittel im radial komprimierten Zustand innerhalb des proximalen Auslassabschnitts so angeordnet ist, dass es in erfindungsgemässe Weise in distaler Richtung zur Längsachse des Instrumentationsstabs hin umgebogen ist. Zudem erleichtert die konische Verjüngung das Überführen des Reinigungsmittels aus dem Depotabschnitt in den Auslassabschnitt. Ferner kann die konische Verjüngung des Innendquerschnitts des Auslassabschnitts in vorteilhafter Weise als mechanischer Anschlag für den Instrumentationsstab dienen. Hierdurch kann die Eindringtiefe des proximalen Endabschnitts des Instrumentationsstabs und der Reinigungsmittel in den Gehörgang technisch besonders einfach begrenzt werden. Hierzu kann der Instrumentationsstab beispielsweise einen radial verbreiterten Abschnitt aufweisen.

Weiterhin ist es vorteilhaft, wenn der Instrumentationsstab elastisch oder flexibel ausgebildet ist. Hierdurch kann sich der Instrumentationsstab beim Einführen dem anatomischen Verlauf des Gehörgangs anpassen, wodurch Verletzungen des Gehörgangs vermieden werden.

Ferner kann es vorgesehen sein, dass der Instrumentationsstab am proximalen Ende des proximalen Endbereichs einen stumpfen oder flächigen Abschluss aufweist. Insbesondere kann der Instrumentationsstab selbst einen stumpfen oder flächigen Abschluss aufweisen. Zusätzlich kann das wenigstens eine Reinigungsmittel so ausgebildet sein, dass es im radial expandierenden Zustand einen im Wesentlichen senkrecht zur Längsachse des Instrumentationsstabs verlaufenden flächigen Abschluss bildet, etwa in Form einer ebenen Kreisflache, die von einem Bürstenkranz gebildet wird. Auch hierdurch werden Verletzungen des Gehörgangs und insbesondere des Trommelfells vermieden.

Die Länge des Instrumentationsstabs entspricht mindestens der maximal gewünschten bzw. anatomisch vorgegebenen Eindringtiefe. Insbesondere kann der Instrumentationsstab eine Austrittslänge von 8 bis 20 mm, vorzugsweise 10-12 mm aufweisen, der aus dem proximalen Austrittsabschnitt der Führungshülse in proximaler Richtung verschiebbar ist. Der Durchmesser des Reinigungsmittels kann zwischen 5 mm und 14 mm, vorzugsweise 10 mm betragen. Dessen Querschnittsform kann zylinderförmig oder ellipsenförmig gemäss der Anatomie des Gehörgangs ausgeführt sein.

Gemäss einer weiteren Ausgestaltung der Erfindung kann der Instrumentationsstab wenigstens einen Innenkanal aufweisen, der im proximalen Endbereich in eine oder mehrere Auslassöffnungen mündet. Der Innenkanal kann in vorteilhafter Weise zum Applizieren von Flüssigkeiten, insbesondere Spül- und/oder Reinigungsflüssigkeiten, von Cremes, Mandelöl oder dergleichen verwendet werden. Flüssigkeiten und Cremes lassen sich zudem in vorteilhafter Weise in dem oben beschrieben Depotabschnitt lagern, um von dort über den wenigstens einen Innenkanal in den Gehörgang appliziert und mit Hilfe des Reinigungsmittels, etwa einer Bürste, im Gehörgang verteilt zu werden.

Ferner kann die Vorrichtung an einem distalen Ende oder im Anschluss an ein distales Ende des der Instrumentationsstabs einen Griff zum Bestätigen des Instrumentationsstabs aufweisen.

Die erfindungsmässe Reinigungsvorrichtung ist bevorzugt aus Kunststoff hergestellt. Insbesondere kann die Reinigungsvorrichtung als Einweg-Vorrichtung ausgebildet sein. Hierzu kann es von Vorteil sein, wenn die Vorrichtung aus biologisch abbaubarem Material, beispielsweise Bioplastik, hergestellt ist.

Die erfindungsmässe Vorrichtung ist vornehmlich zur Reinigung des äusseren Gehörgangs beim Menschen bestimmt, kann jedoch aber auch zur Reinigung des äusseren Gehörgangs von Tieren, etwa von Hunden oder Katzen, verwendet werden.

Gemäss einer besonders bevorzugten Ausführungsform ist die gesamte Vorrichtung in Form einer Einweg-Spritze ausgebildet. Dabei ist ein Spritzenzylinder vorzugsweise durch einen distalen, insbesondere zylinderförmigen Depotabschnitt der erfindungsgemässen Führungshülse gebildet (insbesondere wie oben beschrieben), der zur Aufnahme des wenigstens einen Reinigungsmittels im radial expandierten Zustand dient. Anschliessend an den Depotabschnitt weist der Spritzenzylinder einen erfindungsgemässen proximalen Auslassabschnitt auf. Der Auslassabschnitt weist vorzugsweise eine Verjüngung des Innen- und/oder Aussenquerschnitts auf, beispielsweise eine hyperbolische, parabolische oder konische bzw. hyperbolisch, parabolisch oder konisch ausgebildete Verjüngung des Aussenquerschnitts und/oder eine hyperbolische, parabolische oder konische bzw. hyperbolisch, parabolisch oder konisch ausgebildete Verjüngung des Innenquerschnitts. Ein Spritzenkolben ist gleitend verschiebbar im Spritzenzylinder geführt. Der Spritzenkolben weist an seinem proximalen Ende eine Zylinderscheibe zur gleitenden, vorzugsweise dichtend gleitenden Führung des Spritzenkolbens im Spritzenzylinder auf. Von der Zylinderscheibe aus erstreckt sich in proximaler Richtung entlang der Kolbenachse ein erfindungsgemässer Instrumentationsstab mit Reinigungseinrichtung. Die Zylinderscheibe dient zudem vorzugsweise als Begrenzer für die Einführtiefe des Instrumentationsstabs, indem sie beim Vorschieben in proximaler Richtung in Anschlag mit der konischen Verjüngung des Innenquerschnitts des proximalen Auslassabschnitts gelangt. Ein weiterer Begrenzer für die Einführtiefe ist durch eine radial verbreiterte Auflage am distalen Ende des Spritzenkolbens gegeben. Diese dient vorzugsweise als Auflage für den Daumen, mit dem der Kolben in proximaler Richtung zum Einführen des Instrumentationsstabs aus dem Depotabschnitt über den Auslassabschnitt in den Gehörgang vorschieben lässt. Beim Vorschieben in proximaler Richtung gelangt die radial verbreiterte Auflage mit dem distalen Ende des Spritzenkolbens in Anschlag. Am distalen Ende des Spritzenzylinders sind bevorzugt zwei sich gegenüberliegende jeweils in radialer Richtung erstreckende Flügel vorgesehen, die beim Vorschieben des Kolbens zur Gegenlagerung der Vorschubkraft als Auflage für Zeige- und Mittelfinger dienen.

Anstelle einer Auflage für den Daumen - wie zuvor beschreiben - sind auch andere Mittel denkbar, um den Instrumentationsstab bzw. den Spritzenkolben zu bedienen. So kann die Vorrichtung vorzugsweise am distalen Ende des Spritzenkolbens bzw. des Instrumentationsstabs einen Griff aufweisen, der beispielsweise mit Daumen und Zeigefinger ergriffen werden kann, um den Spritzenkolben bzw. den Instrumentationsstab zu betätigen. Die Verwendung eines solchen Griffs zeichnet sich insbesondere dadurch aus, dass sich der Spritzenkolben bzw. der Instrumentationsstab darüber gut kontrolliert nicht nur in proximaler Richtung vorschieben, sondern im Vergleich zu einer Auflage für den Daumen auch wesentlich einfacher in distaler Richtung zurückziehen lässt. Auch bei Verwendung eines solchen Griffs kann am distalen Ende des Spritzenkolbens vorzugsweise eine radial verbreiterte Auflage vorgesehen sein, die als Anschlag mit dem distalen Ende des Spritzenzylinders zur Begrenzung der Einführtiefe dient. In dieser Konfiguration kann sich der Griff in distaler Richtung an die radial verbreiterte Auflage anschliessen. Vorzugsweise ist der Griff am distalen Ende des Spritzenkolbens, insbesondere - sofern vorhanden - an die radial verbreiterte Auflage angespritzt.

Ein weiterer Aspekt der Erfindung betrifft ein Verfahren zum Reinigen des äusseren Gehörgangs unter Verwendung einer erfindungsgemässen Vorrichtung, insbesondere wie zuvor beschrieben. Das Verfahren ist durch folgende Schritte gekennzeichnet:
a. Einführen des proximalen Auslassabschnitts der Führungshülse in den äusseren Gehörgang bis zu einer vorbestimmten Einführtiefe in einer ersten Vorschubbewegung;
b. Vorschieben des Instrumentationsstabs in proximaler Richtung durch die Führungshülse über den proximalen Auslassabschnitt in den äusseren Gehörgang in einer zweiten Vorschubbewegung, bis das wenigstens eine Reinigungsmittel aus dem komprimierten Zustand in den radial expandierten Zustand übergangen ist; und
c. Gleichzeitiges Zurückziehen der Führungshülse und des Instrumentationsstabs im radial expandierten Zustand des Reinigungsmittels aus dem äusseren Gehörgang in einer einzigen gemeinsamen Rückzugbewegung.

Die Erfindung betrifft zudem ein weiteres Verfahren zum Reinigen des äusseren Gehörgangs unter Verwendung einer erfindungsgemässen Vorrichtung, insbesondere wie zuvor beschrieben. Das weitere Verfahren ist durch folgende Schritte gekennzeichnet:
a. In einer einzigen gemeinsamen Vorschubbewegung: Einführen des proximalen Auslassabschnitts der Führungshülse in den äusseren Gehörgang bis zu einer vorbestimmten Einführtiefe und gleichzeitiges Vorschieben des Instrumentationsstabs in proximaler Richtung durch die Führungshülse über den proximalen Auslassabschnitt in den äusseren Gehörgang, bis das wenigstens eine Reinigungsmittel aus dem komprimierten Zustand in den radial expandierten Zustand übergangen ist; und
b. Gleichzeitiges Zurückziehen der Führungshülse und des Instrumentationsstabs im radial expandierten Zustand des Reinigungsmittels aus dem äusseren Gehörgang in einer einzigen gemeinsamen Rückzugbewegung.

Weitere Ziele, Vorteile, Merkmale und Anwendungsmöglichkeiten der vorliegenden Erfindung ergeben sich aus der nachfolgenden Beschreibung eines in den Zeichnungen gezeigten Ausführungsbeispieles. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen oder deren Rückbeziehung.

Es zeigt:
- Fig. 1: ein Ausführungsbeispiel einer erfindungsgemässen Vorrichtung zum Reinigen des äusseren Gehörgangs in perspektivischer Darstellung;
- Fig. 2: das Ausführungsbeispiel der erfindungsgemässen Vorrichtung gemäss Fig. 1 im Längsschnitt;
- Fig. 3: das Ausführungsbeispiel der erfindungsgemässen Vorrichtung gemäss Fig. 1 im Längsschnitt vor der Verwendung; und
- Fig. 4: ein weiteres Ausführungsbeispiel einer erfindungsgemässen Vorrichtung mit einem Griff.

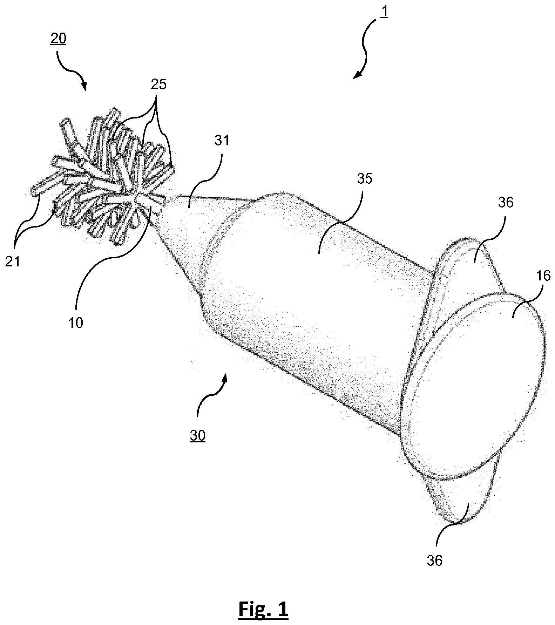

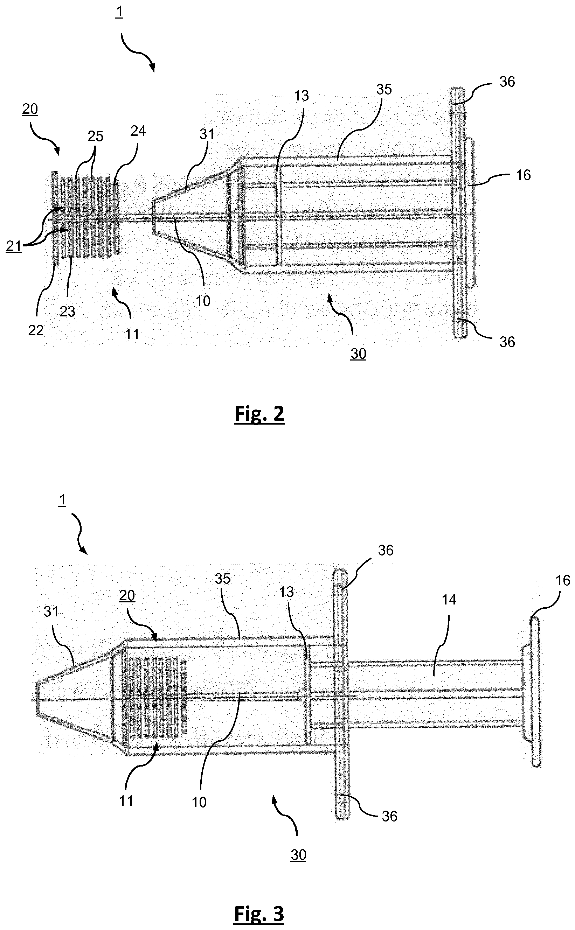

Die Fig. 1-3 zeigen ein mögliches Ausführungsbeispiel einer erfindungsgemässen Vorrichtung 1 zum Reinigen des äusseren Gehörgangs. Gemäss der Erfindung umfasst die Vorrichtung 1 einen Instrumentationsstab 10, an dessen proximalem Endbereich 11 eine Reinigungseinrichtung 20 mit mehreren umfangsseitig verlaufenden in radialer Richtung elastisch expandierenden Reinigungsmitteln 21 angeordnet ist. Im vorliegenden Ausführungsbespiel ist die Reinigungseinrichtung 20 eine Bürste zum Reinigen des äusseren Gehörgangs. Wie insbesondere den Fig. 2 und 3 zu entnehmen ist, weist die Bürste vorliegend mehrere Reinigungsmitteln 21 in Form mehrerer - hier beispielsweise neun - Bürstenkränze 22, 23, 24 auf, die am Instrumentationsstab 10 in axialer Richtung (bezüglich Längsachse des Instrumentationsstabs 10) entlang des proximalen Endbereichs 11 verteilt angeordnet sind. Jeder Bürstenkranz 22, 23, 24 besteht aus mehreren - hier beispielsweise fünf - umfangsseitig angeordneten Borsten 25, die sich im frei expandierten Zustand vom Instrumentationsstab 10 ausgehend radial senkrecht zu dessen Längsachse nach aussen erstrecken. Insbesondere sind sämtliche Borsten 25 eines Bürstenkranzes 22, 23, 24 gleich lang und in einer Axialebene bezüglich der Längsachse des Instrumentationsstabs 10 angeordnet, so dass sie eine kreisförmige Bürstenscheibe bilden. Wie insbesondere Fig. 1 zeigt, sind die mehreren axial voneinander beanstandeten Bürstenkränze 22, 23, 24 so angeordnet, dass die Borsten benachbarter Bürstenkränze umfangsseitig versetzt zueinander angeordnet sind. Die Borsten 25 sind bevorzugt Kunststoffborsten. Auch der Instrumentationsstab besteht bevorzugt aus Kunststoff, insbesondere aus einem flexiblen Kunststoff, so dass er sich beim Einführen in den Gehörgang dem anatomischen Verlauf des Ohrkanals gut anpasst. Das proximale Ende des Endbereichs 11 bildet zusammen mit dem in proximaler Richtung zuvorderst angeordneten Bürstenkranz 22 einen stumpfen bzw. flächigen Abschluss, wodurch in vorteilhafter Weise Verletzungen des Gehörgangs und insbesondere des Trommelfells vermieden werden. Die Borsten können einseitig mit dem Instrumentationsstab 10 gespritzt sein.

Gemäss der Erfindung umfasst die Vorrichtung 1 ferner eine Führungshülse 30 mit einem proximalen Auslassabschnitt 31 zur partiellen Einführung in den äusseren Gehörgang, durch die hindurch der Instrumentationsstab 10 samt Reinigungsvorrichtung 20 in den Gehörgang einbringbar ist. Zusätzlich zum proximalen Auslassabschnitt 31 weist die Führungshülse 30 einen distalen, zylinderförmigen Depotabschnitt 35 auf, der zur Aufnahme und Aufbewahrung der bürstenartigen Reinigungseinrichtung 20 im radial frei expandierten, d.h. entspannten Zustand dient. Damit kann einem Verlust der elastischen Materialeigenschaften der Borsten zwischen Produktion und eigentlicher Verwendung der Reinigungsvorrichtung vorgebeugt werden. Der Innenquerschnitt des zylindrischen Depotabschnitts 35 ist hierzu grösser ausgebildet als der grösste Aussenquerschnitt der Bürstenkränze 22, 23, 24 im radial expandierten Zustand. Gleichzeitig wird die Bürste von dem umhüllenden Depotabschnitt 35 vor schädigenden Ausseneinflüssen, etwa Verschmutzungen, geschützt.

Wie den Fig. 2 und 3 im Detail zu entnehmen ist, ist der proximale Auslassabschnitt 31 im vorliegenden Ausführungsbeispiel in Form eines Hohlkegelstumpfs ausgebildet, der sich in proximaler Richtung an den zylinderförmigen Depotabschnitt 35 anschliesst. Sowohl der Innenquerschnitt als auch der Aussenquerschnitt des Auslassabschnitts 31 nehmen in proximaler Richtung konisch ab. Denkbar ist auch eine hyperbolisch oder parabolisch ausgebildete Verjüngung. Dabei bewirkt die gewählte Geometrie, beispielsweise vorliegend der Aussenkonus, dass die Eindringtiefe des Auslassabschnitts 31 je nach Weite des Gehörgangs automatisch begrenzt ist und damit das Verletzungsrisiko reduziert wird. Zudem wird durch den Aussenkonus ein Anschlag für die Führungshülse beim Ansetzen bzw. partiellen Einführen in den Gehörgang bewirkt, was die Handhabbarkeit Vorrichtung 1 insgesamt erleichtert. Insbesondere kann die Vorrichtung hierdurch einhändig vorgenommen werden.

Der Innenquerschnitt des Auslassabschnitts 31 dient gemäss der Erfindung dazu, die Bürstenkränze 22, 23, 24 beim Vorschieben des Instrumentationsstabs in proximaler Richtung aus dem Depotabschnitt, in dem sie sich im frei expandierten, entlasteten Zustand befinden, radial zu komprimieren, d.h. in einen radial komprimierten Zustand zu bringen. In erfindungsgemässe Weise erfolgt das Komprimieren derart, dass die Borsten 25 sämtlicher Bürstenkränze 22, 23, 24 innerhalb des proximalen Auslassabschnitts 31 in distaler Richtung zur Längsachse des Instrumentationsstabs 10 hin umgebogen sind, d.h. mit ihren freien Enden von der proximalen Ausgangsöffnung des Konus weg in Richtung des sich öffnenden Konus zeigen. Dabei erleichtert die trichterförmige, insbesondere konische Verjüngung des Innenquerschnitts das Überführen und Komprimieren der Bürstenkränze 22, 23, 24 aus dem Depotabschnitt 35 in den Auslassabschnitt 31. Ferner kann die konische Verjüngung des Innendquerschnitts in vorteilhafter Weise als mechanischer Anschlag für eine radiale Verbreiterung 13 an einem distalen Endbereich des Instrumentationsstabs 10 dienen. Wie die Fig. 2 und 3 im Detail zeigen ist, weist der kleinste Innenquerschnitt des proximalen Auslassabschnitts 31 im Bereich der proximalen Auslassöffnung der konischen Auslassabschnitts 31 einen deutlich kleineren Durchmesser auf, als der Aussenquerschnitt der bürstenartigen Reinigungseinrichtung 20 im frei expandierten Zustand. Der Durchmesser der Auslassöffnung des konischen Auslassabschnitts 31 und damit des Durchmesser des Aussenquerschnitts der bürstenartigen Reinigungseinrichtung 20 im radial komprimierten Zustand beträgt im vorliegenden Ausführungsbespiel etwa 4 mm und ist daher kleiner als der Innenquerschnitt des Gehörgangs, der bei erwachsenen Personen typischerweise im Bereich von etwa 4 - 9 mm, durchschnittlich bei etwa 7 mm liegt. Somit kann die bürstenartigen Reinigungseinrichtung 20 durch Vorschieben des Instrumentationsstabs 10 zumindest partiell in den äussern Gehörgang eingeführt werden, ohne dass sie an die Innenwandung des Gehörgangs gelangt und ungewollt Cerumen in proximaler Richtung vorschiebt.

Dadurch, dass die Borsten 25 der Bürstenkränze 22, 23, 24 innerhalb des proximalen Auslassabschnitts 31 im radial komprimierten Zustand in distaler Richtung zur Längsachse des Instrumentationsstabs 10 hin umgebogen sind, wird insbesondere erreicht, dass sich die Borsten beim Austritt aus dem proximalen Auslassabschnitt 31 in den radial expandierten Zustand in proximaler Richtung, d.h. zum Ohrinneren hin bzw. in Richtung Trommelfell, aufrichten. Wie insbesondere Fig. 2 zu entnehmen ist, sind die Borsten 25 des in proximaler Richtung zuvorderst angeordneten Bürstenkranzes 22 länger als die Borsten 25 des zuhinterst angeordneten Bürstenkranzes 24. Die Länge der Borsten 25 der dazwischenliegenden Bürstenkränze 23 liegt betragsmässig zwischen der Länge der Borsten 25 der zuvorderst und zuhinterst angeordneten Bürstenkränze 22, 24. Die Bürste weist somit im radial expandierten Zustand einen in proximaler Richtung konisch oder gestuft zunehmende Aussenquerschnitt auf. Hierdurch wird erreicht, dass sich sämtliche Reinigungsmittel in den expandierten Zustand erst aufrichten, wenn das entlang des proximalen Endabschnitts proximal zuvorderst angeordnete Reinigungsmittel vollständig aus dem proximalen Austrittsabschnitts ausgetreten ist. In vorteilhafter Weise kann hierdurch eine deutlicher grössere Einführtiefe des Reinigungsmittels in proximaler Richtung erzielt werden, ohne dass dabei Cerumen durch das (noch komprimierte) Reinigungsmittel in proximaler Richtung vorgeschoben oder verdichtet wird. Letzteres wird insbesondere dadurch vermieden, dass das Reinigungsmittel erst bei Erreichen der maximal gewünschten Eindringtiefe des Instrumentationsstabs 10 in den Gehörgang in den radial expandierten Zustand und damit gegebenenfalls in Kontakt mit der Innenwandung des Gehörgangs gelangt.

Bei dem in den Fig. 1-3 gezeigten Ausführungsbespiel ist die gesamte Vorrichtung 1 in Form einer Einweg-Spritze ausgebildet, die sich durch eine besonders leichte Handhabbarkeit auszeichnet. Ein Spritzenzylinder ist durch den zylinderförmigen Depotabschnitt 35 gebildet, an den sich der konische Auslassabschnitt 31 in proximaler Richtung als eine Art Auslassdüse oder Auslasstrichter anschliesst. Ein Spritzenkolben 14 ist mittels einer Zylinderscheibe 13 gleitend verschiebbar im Spritzenzylinder geführt. Von der Zylinderscheibe 13 aus erstreckt sich in proximaler Richtung entlang der Kolbenachse der Instrumentationsstab 10 mit Reinigungseinrichtung 20. Die Zylinderscheibe 13 dient zudem als Begrenzer für die Einführtiefe des Instrumentationsstabs 20 in den Gehörgang, indem sie beim Vorschieben in proximaler Richtung in Anschlag mit dem Innenkonus des proximalen Auslassabschnitts 31 gelangt. Ein weiterer Begrenzer der Einführtiefe ist durch eine radial verbreiterte Auflage 16 am distalen Ende des Spitzenkolbens gegeben, die vorzugsweise als Auflage für den Daumen zum Vorschieben des Kolbens 14 in proximaler Richtung dient. Beim Vorschieben in proximaler Richtung gelangt die radial verbreiterte Auflage 16 mit dem distalen Ende des Spritzenkolbens in Anschlag. Am distalen Ende des Spritzenzylinders sind zudem zwei sich gegenüberliegende jeweils in radialer Richtung erstreckende Flügel 36 vorgesehen, die beim Vorschieben des Kolbens zur Gegenlagerung der Vorschubkraft als Auflage für Zeige- und Mittelfinger dienen.

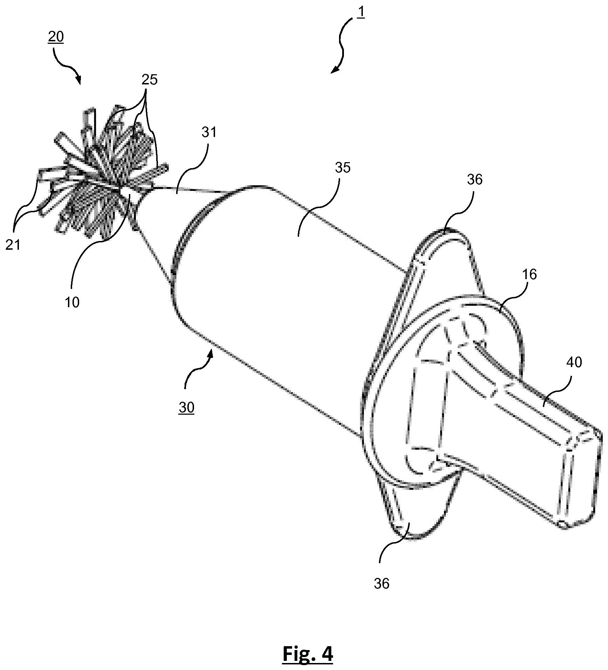

Anstelle einer Auflage für den Daumen - wie zuvor beschrieben - sind auch andere Mittel denkbar, um den Instrumentationsstab bzw. den Spritzenkolben 14 zu bedienen. So kann die Vorrichtung 1 gemäss einem weiteren Ausführungsbespiel der Erfindung - wie in Fig. 4 gezeigt - am distalen Ende des Spritzenkolbens 14 einen Griff 40 aufweisen, der beispielsweise mit Daumen und Zeigefinger ergriffen werden kann, um den Spritzenkolben 14 zu betätigen. Die Verwendung eines solchen Griffs 40 zeichnet sich insbesondere dadurch aus, dass sich der Spritzenkolben 14 darüber gut kontrolliert nicht nur in proximaler Richtung vorschieben, sondern im Vergleich zu einer Auflage für den Daumen auch wesentlich einfacher in distaler Richtung zurückziehen lässt. Auch bei Verwendung eines solchen Griffs 40 kann am distalen Ende des Spritzenkolbens 14 vorzugsweise eine radial verbreiterte Auflage 16 vorgesehen sein, die als Anschlag mit dem distalen Ende des Spritzenzylinders zur Begrenzung der Einführtiefe dient. In dieser Konfiguration kann sich der Griff 40 in distaler Richtung an die radial verbreiterte Auflage 16 anschliessen. Vorzugsweise ist der Griff 40 am distalen Ende des Spritzenkolbens 14, insbesondere - sofern vorhanden - an die radial verbreiterte Auflage 16 angespritzt.

Die Reinigung des Gehörgangs kann mithilfe der vorliegend gezeigten Ausführungsbeispiele der Vorrichtung grundsätzlich in wenigstens zwei Varianten erfolgen. In einer ersten Variante wird die gesamte Reinigungsvorrichtung 1 in einer einzigen Bewegung in den Gehörgang eingeführt und in einer einzigen Bewegung wieder aus dem Gehörgang herausgezogen. Insbesondere können in einer einzigen gemeinsamen Vorschubbewegung die Führungshülse 30 mit dem proximalen Auslassabschnitt 31 in den äusseren Gehörgang bis zu einer vorbestimmten Einführtiefe eingeführt und gleichzeitig durch Vorschieben der Instrumentationsstabs 10 in proximaler Richtung die bürstenartige Reinigungseinrichtung 20 aus dem Depotabschnitt 35 über den proximalen Auslassabschnitt 31 in den äusseren Gehörgang eingeführt werden, bis die Borsten 25 der Bürstenkränze 22, 23, 24 aus dem vorübergehend komprimierten Zustand in den radial expandierten Zustand übergangen sind. Sodann kann die Führungshülse 30 und der Instrumentationsstab 10 im radial expandierten Zustand der Bürstenkränze 22, 23, 24 in einer einzigen gemeinsamen Rückzugbewegung aus dem äusseren Gehörgang herausgezogen werden, wobei etwaig in distaler Richtung vorhandenes Cerumen aus dem Gehörgang herausgefördert wird.

In einer zweiten Variante wird zunächst nur die Führungshülse 30 mit dem proximalen Auslassabschnitt 31 in einer ersten Vorschubbewegung bis zu einer vorbestimmten Einführtiefe in den äusseren Gehörgang eingeführt. Dabei befindet sich die die bürstenartige Reinigungseinrichtung 20 vorzugsweise noch im Depotabschnitt 35. In einer zweiten Vorschubbewegung wird sodann durch Vorschieben des Instrumentationsstabs 10 in proximaler Richtung die bürstenartige Reinigungseinrichtung 20 aus dem Depotabschnitt 35 über den proximalen Auslassabschnitt 31 in den äusseren Gehörgang eingeführt, bis die Borsten 25 der Bürstenkränze 22, 23, 24 aus dem vorübergehend komprimierten Zustand in den radial expandierten Zustand übergangen sind. Anschliessend werden dann - wie bei der ersten Variante - die Führungshülse 30 und der Instrumentationsstab 10 im radial expandierten Zustand der Borsten 25 in einer einzigen gemeinsamen Rückzugbewegung aus dem äusseren Gehörgang herausgezogen.

## Patentansprüche

1. Vorrichtung (1) zum Reinigen des äusseren Gehörgangs, umfassend eine Führungshülse (30) mit einem proximalen Auslassabschnitt (31) zur partiellen Einführung in den Gehörgang und einen axial durch die Führungshülse (30) hindurchführbaren Instrumentationsstab (10), welcher an einem proximalen Endbereich (11) eine Reinigungseinrichtung (20) mit wenigstens einem umfangsseitig verlaufenden, in radialer Richtung elastisch expandierenden Reinigungsmittel (21) aufweist, **dadurch gekennzeichnet, dass** ein Innenquerschnitt des proximalen Auslassabschnitts (31) kleiner ist als ein Aussenquerschnitt des Reinigungsmittels (21) in einem radial expandierten Zustand, wobei das wenigstens eine Reinigungsmittel (21) innerhalb des proximalen Auslassabschnitts (31) in einem radial komprimierten Zustand anordenbar ist, in welchem das Reinigungsmittel (21) in distaler Richtung zur Längsachse des Instrumentationsstabs (10) hin umgebogen ist, so dass sich das wenigstens eine Reinigungsmittel (21) beim Austritt aus dem proximalen Auslassabschnitt (31) in proximaler Richtung in den radial expandierten Zustand aufrichtet.

2. Vorrichtung (1) nach Anspruch 1, **dadurch gekennzeichnet, dass** das Reinigungsmittel (21) wenigstens einen umlaufenden Bürstenkranz (22, 23, 24) aus mehreren umfangsseitig angeordneten Borsten (25), flexiblen Scheiben oder Kreissegmenten, beispielsweise aus Kunststoff aufweist, die sich im radial frei expandierten Zustand vom Instrumentationsstab (10) quer zur Längsachse nach aussen erstrecken.

3. Vorrichtung (1) nach Anspruch 1 oder 2, **dadurch gekennzeichnet, dass** die Reinigungseinrichtung (20) mehrere Reinigungsmittel (21), insbesondere mehrere Bürstenkränze (22, 23, 24), flexible Scheiben oder Kreissegmente, beispielsweise aus Kunststoff aufweist, die axial verteilt entlang des proximalen Endabschnitts (11) angeordnet sind.

4. Vorrichtung (1) nach Anspruch 3, **dadurch gekennzeichnet, dass** der Aussenquerschnitt der Reinigungseinrichtung (20), insbesondere die radialen Abmessungen der mehreren Reinigungsmittel (21) im radial expandierten Zustand entlang des proximalen Endabschnitts (11) in distaler Richtung abnimmt.

5. Vorrichtung (1) nach einem der vorherigen Ansprüche, **dadurch gekennzeichnet, dass** die Führungshülse (30), insbesondere der Auslassabschnitt (31) in proximaler Richtung eine vorzugsweise konische, hyperbolische oder parabolische Verjüngung des Innenquerschnitts aufweist.

6. Vorrichtung (1) nach einem der vorherigen Ansprüche, **dadurch gekennzeichnet, dass** die Führungshülse (30), insbesondere der Auslassabschnitt (31) in proximaler Richtung eine vorzugsweise konische Verjüngung des Aussenquerschnitts aufweist.

7. Vorrichtung (1) nach einem der vorherigen Ansprüche, **dadurch gekennzeichnet, dass** die Führungshülse (30) einen distalen, vorzugsweise zylinderförmigen Depotabschnitt (35) zur Aufnahme des wenigstens einen Reinigungsmittels (21) im radial expandierten Zustand aufweist, wobei ein kleinster Innenquerschnitt des distalen Depotabschnitts grösser ist als der grösste Aussenquerschnitt des proximalen Endbereichs (11) im radial expandierten Zustand des Reinigungsmittels (21).

8. Vorrichtung (1) nach einem der vorherigen Ansprüche, **dadurch gekennzeichnet, dass** der Instrumentationsstab (10) elastisch ausgebildet ist.

9. Vorrichtung (1) nach einem der vorherigen Ansprüche, **dadurch gekennzeichnet, dass** der Instrumentationsstab (10) wenigstens einen Innenkanal aufweist, der im proximalen Endbereich (11) in eine oder mehrere Auslassöffnungen mündet.

10. Vorrichtung (1) nach einem der vorherigen Ansprüche, **dadurch gekennzeichnet, dass** der Instrumentationsstab (10) am proximalen Ende des proximalen Endbereichs (11) einen stumpfen oder flächigen Abschluss aufweist.

11. Vorrichtung (1) nach einem der vorherigen Ansprüche, **dadurch gekennzeichnet, dass** die Vorrichtung an einem distalen Ende oder im Anschluss an ein distales Ende des der Instrumentationsstabs (10) einen Griff zum Bestätigen des Instrumentationsstabs (10) aufweist.

12. Verfahren zum Reinigen des äusseren Gehörgangs unter Verwendung einer Vorrichtung (1) nach einem der vorherigen Ansprüche, **gekennzeichnet durch** folgende Schritte:
a. Einführen des proximalen Auslassabschnitts (31) der Führungshülse (30) in den äusseren Gehörgang bis zu einer vorbestimmten Einführtiefe in einer ersten Vorschubbewegung;
b. Vorschieben des Instrumentationsstabs (10) in proximaler Richtung durch die Führungshülse (30) über den proximalen Auslassabschnitt (31) in den äusseren Gehörgang in einer zweiten Vorschubbewegung, bis das wenigstens eine Reinigungsmittel (21) aus dem komprimierten Zustand in den radial expandierten Zustand übergegangen ist; und
c. Gleichzeitiges Zurückziehen der Führungshülse (30) und des Instrumentationsstabs (10) im radial expandierten Zustand des Reinigungsmittels (21) aus dem äusseren Gehörgang in einer einzigen gemeinsamen Rückzugbewegung.

13. Verfahren zum Reinigen des äusseren Gehörgangs unter Verwendung einer Vorrichtung (1) nach einem der vorherigen Ansprüche, **gekennzeichnet durch** folgende Schritte:
a. In einer einzigen gemeinsamen Vorschubbewegung: Einführen des proximalen Auslassabschnitts (31) der Führungshülse (30) in den äusseren Gehörgang bis zu einer vorbestimmten Einführtiefe und gleichzeitiges Vorschieben des Instrumentationsstabs (10) in proximaler Richtung durch die Führungshülse (30) über den proximalen Auslassabschnitt (31) in den äusseren Gehörgang, bis das wenigstens eine Reinigungsmittel (21) aus dem komprimierten Zustand in den radial expandierten Zustand übergegangen ist; und
b. Gleichzeitiges Zurückziehen der Führungshülse (30) und des Instrumentationsstabs (10) im radial expandierten Zustand des Reinigungsmittels (21) aus dem äusseren Gehörgang in einer einzigen gemeinsamen Rückzugbewegung.

## Claims

1. A device (1) for cleaning the external auditory meatus, comprising a guide sleeve (30) with a proximal outlet portion (31) for partial insertion into the auditory meatus and an instrumentation rod (10) which can be axially passed through the guide sleeve (30) and which has, at a proximal end region (11), a cleaning apparatus (20) with at least one cleaning means (21) which runs circumferentially and expands elastically in the radial direction,
**characterized in that**
an inner cross-section of the proximal outlet portion (31) is smaller than an outer cross-section of the cleaning means (21) in a radially expanded state, wherein the at least one cleaning means (21) can be arranged within the proximal outlet portion (31) in a radially compressed state in which the cleaning means (21) is bent towards the longitudinal axis of the instrumentation rod (10) in the distal direction, such that the at least one cleaning means (21) becomes upright while changing to the radially expanded state when it moves out of the proximal outlet portion (31) in the proximal direction.

2. The device (1) according to Claim 1, **characterized in that** the cleaning means (21) has at least one circumferential brush ring (22, 23, 24) of several circumferentially arranged brushes (25), flexible discs or circular segments, for example made of plastics, which extend outwardly from the instrumentation rod (10) perpendicularly to the longitudinal axis in the radially freely expanded state.

3. The device (1) according to Claim 1 or 2, **characterized in that** the cleaning apparatus (20) has several cleaning means (21), in particular several brush rings (22, 23, 24), flexible discs or circular segments, for example made of plastics, which are arranged to be axially distributed along the proximal end portion (11).

4. The device (1) according to Claim 3, **characterized in that** the outer cross-section of the cleaning apparatus (20), in particular the radial dimensions of the several cleaning means (21), decrease in the distal direction along the proximal end portion (11) in the radially expanded state.

5. The device (1) according to any one of the preceding claims, **characterized in that** the guide sleeve (30), in particular the outlet portion (31), has a preferably conical, hyperbolic or parabolic tapering of the inner cross-section in the proximal direction.

6. The device (1) according to any one of the preceding claims, **characterized in that** the guide sleeve (30), in particular the outlet portion (31), has a preferably conical tapering of the outer cross-section in the proximal direction.

7. The device (1) according to any one of the preceding claims, **characterized in that** the guide sleeve (30) has a distal, preferably cylindrical reservoir portion (35) for receiving the at least one cleaning means (21) in the radially expanded state, wherein a smallest inner cross-section of the distal reservoir portion is larger than the largest outer cross-section of the proximal end portion (11) in the radially expanded state of the cleaning means (21).

8. The device (1) according to any one of the preceding claims, **characterized in that** the instrumentation rod (10) is designed to be elastic.

9. The device (1) according to any one of the preceding claims, **characterized in that** the instrumentation rod (10) has at least one inner channel leading into one or more outlet openings in the proximal end region (11).

10. The device (1) according to any one of the preceding claims, **characterized in that** the instrumentation rod (10) has a dull or flat ending at the proximal end of the proximal end region (11).

11. The device (1) according to any one of the preceding claims, **characterized in that** the device has a hilt for actuating the instrumentation rod (10) at a distal end or connected to a distal end of the instrumentation rod (10).

12. A method for cleaning the external auditory meatus using a device (1) according to any one of the preceding claims, **characterized by** the following steps:
a. inserting the proximal outlet portion (31) of the guide sleeve (30) into the external auditory meatus up to a predetermined insertion depth in a first advancing movement;
b. advancing the instrumentation rod (10) in the proximal direction through the guide sleeve (30) via the proximal outlet portion (31) into the external auditory meatus in a second forward motion until the at least one cleaning means (21) has changed from the compressed state to the radially expanded state; and
c. simultaneous withdrawal of the guide sleeve (30) and the instrumentation rod (10) in the radially expanded state of the cleaning means (21) from the external auditory meatus in a single, combined withdrawal movement.

13. A method for cleaning the external auditory meatus using a device (1) according to any one of the preceding claims, **characterized by** the following steps:
a. in a single, combined advancing movement: inserting the proximal outlet portion (31) of the guide sleeve (30) into the external auditory meatus up to a predetermined insertion depth and simultaneously advancing the instrumentation rod (10) in the proximal direction through the guide sleeve (30) via the proximal outlet portion (31) into the external auditory meatus until the at least one cleaning means (21) has changed from the compressed state to the radially expanded state; and
b. simultaneous withdrawal of the guide sleeve (30) and the instrumentation rod (10) in the radially expanded state of the cleaning means (21) from the external auditory meatus in a single, combined withdrawal movement.

## Revendications

1. Dispositif (1) de nettoyage du conduit auditif externe, comprenant une gaine de guidage (30) avec une section de sortie proximale (31) pour une introduction partielle dans le conduit auditif et un bâtonnet d'instrumentation (10) pouvant traverser axialement la gaine de guidage (30), lequel présente, dans une zone d'extrémité proximale (11), un dispositif de nettoyage (20) comportant au moins un moyen de nettoyage (21) à extension périphérique et élastiquement expansible radialement,
**caractérisé en ce que** la section transversale intérieure de la section de sortie proximale (31) est inférieure à la section transversale extérieure du moyen de nettoyage (21) en état d'expansion radiale, ledit au moins un moyen de nettoyage (21) pouvant être disposé dans la section de sortie proximale (31) en état de compression radiale dans lequel le moyen de nettoyage (21) est replié en direction distale par rapport à l'axe longitudinal du bâtonnet d'instrumentation (10), de sorte que ledit au moins un moyen de nettoyage (21) en sortant de la section de sortie proximale (31) se redresse en direction proximale vers l'état d'expansion radiale.

2. Dispositif (1) selon la revendication 1, **caractérisé en ce que** le moyen de nettoyage (21) présente au moins une couronne de brossage (22, 23, 24) périphérique constituée de plusieurs brins (25), disques flexibles ou segments circulaires, notamment en matière plastique, s'étendant perpendiculairement à l'axe longitudinal vers l'extérieur depuis le bâtonnet d'instrumentation (10) en état de libre expansion radiale.

3. Dispositif (1) selon la revendication 1 ou la revendication 2, **caractérisé en ce que** le dispositif de nettoyage (20) comporte plusieurs moyens de nettoyage (21), notamment plusieurs couronnes de brossage (22, 23, 24), des disques flexibles ou des segments circulaires, notamment en matière plastique, répartis axialement le long de la section d'extrémité proximale (11).

4. Dispositif (1) selon la revendication 3, **caractérisé en ce que** la section transversale extérieure du dispositif de nettoyage (20), notamment les dimensions radiales de la pluralité de moyens de nettoyage (21) décroissent en direction distale le long de la section d'extrémité proximale (11) en état d'expansion radiale.

5. Dispositif (1) selon l'une quelconque des revendications précédentes, **caractérisé en ce que** la gaine de guidage (30), notamment la section de sortie (31) présente un rétrécissement de section intérieure, de préférence conique, hyperbolique ou parabolique en direction proximale.

6. Dispositif (1) selon l'une quelconque des revendications précédentes, **caractérisé en ce que** la gaine de guidage (30), notamment la section de sortie (31) présente en direction proximale un rétrécissement, de préférence conique, de la section transversale extérieure.

7. Dispositif (1) selon l'une quelconque des revendications précédentes, **caractérisé en ce que** la gaine de guidage (30) présente une section de dépôt distale (35), de préférence cylindrique, destinée à recevoir ledit au moins un moyen de nettoyage (21) en état d'expansion radiale, la section transversale intérieure minimale de la section de dépôt distale étant supérieure à la section transversale extérieure maximale de la zone d'extrémité proximale (11) en état d'expansion radiale du produit nettoyant (21).

8. Dispositif (1) selon l'une quelconque des revendications précédentes, **caractérisé en ce que** le bâtonnet d'instrumentation (10) est élastique.

9. Dispositif (1) selon l'une quelconque des revendications précédentes, **caractérisé en ce que** le bâtonnet d'instrumentation (10) présente au moins un canal intérieur débouchant dans une ou plusieurs ouvertures de sortie dans la zone d'extrémité proximale (11).

10. Dispositif (1) selon l'une quelconque des revendications précédentes, **caractérisé en ce que** le bâtonnet d'instrumentation (10) présente une terminaison émoussée ou plate à l'extrémité proximale de la zone d'extrémité proximale (11).

11. Dispositif (1) selon l'une quelconque des revendications précédentes, **caractérisé en ce que** ledit dispositif présente, à l'extrémité distale ou dans le prolongement de l'extrémité distale du bâtonnet d'instrumentation (10), une poignée d'actionnement du bâtonnet d'instrumentation (10).

12. Procédé de nettoyage du conduit auditif externe au moyen d'un dispositif (1) selon l'une quelconque des revendications précédentes, **caractérisé par** les étapes suivantes :
a. introduction de la section de sortie proximale (31) de la gaine de guidage (30) dans le conduit auditif externe jusqu'à une profondeur d'insertion prédéterminée lors d'un premier mouvement d'avance ;
b. refoulement du bâtonnet d'instrumentation (10) au travers de la gaine de guidage (30) en direction proximale, via la section de sortie proximale (31), vers le conduit auditif externe lors d'un deuxième mouvement d'avance, jusqu'à ce que ledit au moins un moyen de nettoyage (21) soit passé de l'état de compression à l'état d'expansion radiale ; et
c. retrait simultané hors du conduit auditif externe de la gaine de guidage (30) et du bâtonnet d'instrumentation (10) en état d'expansion radiale du moyen de nettoyage (21) en un seul mouvement de retrait commun.

13. Procédé de nettoyage du conduit auditif externe au moyen d'un dispositif (1) selon l'une quelconque des revendications précédentes, **caractérisé par** les étapes suivantes :
a. en un seul mouvement d'avance commun : introduction de la section de sortie proximale (31) de la gaine de guidage (30) dans le conduit auditif externe jusqu'à une profondeur d'insertion prédéterminée et refoulement simultané du bâtonnet d'instrumentation (10) en direction proximale au travers de la gaine de guidage (30), via la section de sortie proximale (31) vers le conduit auditif externe, jusqu'à ce que ledit au moins un moyen de nettoyage (21) soit passé de l'état de compression à l'état d'expansion radiale ; et
b. retrait simultané hors du conduit auditif externe de la gaine de guidage (30) et du bâtonnet d'instrumentation (10) en état d'expansion radiale du moyen de nettoyage (21) en un seul mouvement de retrait commun.
